# EUROPEAN PATENT SPECIFICATION

(11) **EP 3 554 307 B1**
(45) Date of publication and mention of the grant of the patent: **17.03.2021**
(21) Application number: 17818101.2
(22) Date of filing: 13.12.2017
(51) Int. Cl.: A45D 2/00, A45D 34/00, A61Q 5/00, A61K 8/892, A61K 8/58, A61K 8/92, A45D 1/00, A45D 1/04, A45D 34/04

(54) **COSMETIC PRODUCT REFILL IN THE FORM OF AN ANHYDROUS COMPOSITION FOR A HAIR TREATMENT DEVICE**
KOSMETISCHE NACHFÜLLUNG IN FORM EINER WASSERFREIEN ZUSAMMENSETZUNG FÜR EINE HAARPFLEGEVORRICHTUNG
PRODUIT COSMÉTIQUE DE REMPLISSAGE SOUS LA FORME D'UNE COMPOSITION ANHYDRE POUR UN DISPOSITIF DE TRAITEMENT CAPILLAIRE

(30) Priority: 15.12.2016 FR 1662504
(43) Date of publication of application: 23.10.2019
(73) Proprietor: L'OREAL, 75008 Paris (FR); SEB S.A., 69130 Ecully (FR)
(72) Inventor: WOODLAND, Frédéric, 93400 Saint-Ouen (FR); CHAMPEAUX, Mélissa, 93500 Saint-Ouen (FR); LAPIZE, Sandy, 93400 Saint-Ouen (FR); VIC, Gabin, 93400 Saint-Ouen (FR); NUZZO, Stefania, 93400 Saint-Ouen (FR)
(74) Representative: Nony
(86) International application number: PCT/EP2017/082539
(87) International publication number: WO 2018/108972

(56) References cited:
- EP-A1- 3 039 988
- WO-A1-2016/189076
- DE-U1-202011 052 113
- FR-A1- 3 015 869

## Description

The present invention relates to cosmetic product refills for hair treatment devices, and more particularly, but not exclusively, those for devices for shaping the hair, notably intended for straightening, curling or crimping the hair.

The invention also relates to devices provided with such a refill and to methods for treating the hair.

### Background

Usually, hair straighteners consist of two arms that are connected together with the aid of a hinge which makes it possible to open and close said arms, and of at least one heating element disposed on the arms. During operations of styling a lock of hair, said lock is introduced between the two arms in the open position and then the two arms are closed manually over the lock of hair. The latter is then subjected to the heat output by the heating element, until the two arms are opened and the lock of hair is removed.

The application WO 2009/078046 describes a hairstyling appliance comprising two arms that are connected together so as to allow the appliance to be opened and closed, at least one heating member and at least one seat for accommodating a hair treatment device, the latter allowing a haircare product to be dispensed during operation. The hair treatment device comprises a support material impregnated with a haircare product and suitable for a single use.

WO 2009/015027 and US 2009/0025247 disclose a hair straightening device that makes it possible to apply a haircare product by contact with the hair. The haircare product to be applied is contained in a removable refill for the application thereof. Said removable refill comprises a reservoir containing the haircare product in a gelled form, and orifices for dispensing and applying the product, said orifices being made directly through a wall of the reservoir. The refill is introduced into a housing disposed on one of the two arms of the hair straightener, by sliding.

The application WO 2013/045331 likewise discloses an applicator for applying a care substance to the hair, comprising a cartridge of care substance held in a housing of the applicator. The cartridge comprises a porous support impregnated with the care substance.

The application WO 2013/090896 relates to a hair treatment appliance, in particular a hairstyling appliance, comprising a treatment agent holder, notably a impregnated porous material, to be applied to the hair or the skin. The holder is fixed to an accessory, which is itself mounted in a removable manner on a hair treatment appliance. The holder is T-shaped, allowing it to be mounted in a complementary groove in the accessory.

There is a need to further improve devices for applying a haircare product so as to ensure adequate application of cosmetic product, notably when the latter is in the form of an anhydrous composition.

### Summary of the invention

A subject of the invention is thus a cosmetic product refill for a hair treatment device, comprising an applicator member impregnated with an anhydrous composition, wherein the applicator member has a density of between 0.11 and 0.25 g/cm³ and the anhydrous composition has a viscosity of between 0.1 and 0.6 Pa.s (100 and 600 centipoise), preferably between 0.1 and 0.5 Pa.s (100 and 500 centipoise).

Under these conditions, homogeneous treatment of the hair is obtained and the cosmetic results are optimum.

Preferably, the cosmetic product refill according to the invention has one or more of the following features, on their own or in combination:
- the applicator member is porous;
- the applicator member has a density of between 0.15 and 0.2 g/cm³ and the anhydrous composition has a viscosity greater than 0.1 Pa.s (100 centipoise), preferably greater than 0.11 Pa.s (110 centipoise) and/or less than 0.12 Pa.s (120 centipoise), preferably less than 0.15 Pa.s (115 centipoise), at 22°C;
- the applicator member is a felt, the fibers of the applicator member preferably being oriented parallel or perpendicularly to the surface intended to be in contact with the hair to be treated, and notably the fibers are oriented in a longitudinal direction of the applicator member, parallel to the surface intended to be in contact with the hair to be treated;
- the applicator member, notably the felt, if appropriate, is made of polyethylene, of polypropylene, or of a mixture of polyethylene and polypropylene, the mixture preferably comprising 50% polyethylene and 50% polypropylene;
- the applicator member is impregnated with anhydrous composition with an initial degree of filling less than or equal to 90%, preferably less than or equal to 80%, more preferably less than or equal to 70%;
- the applicator member is impregnated with anhydrous composition with an initial degree of filling greater than or equal to 40%, preferably greater than or equal to 50%, more preferably greater than or equal to 60%;
- the applicator member is impregnated with anhydrous composition with a degree of filling greater than 40%, preferably greater than 50%, more preferably greater than 60%, and/or less than 90%, preferably less than 85%, more preferably less than 80%;
- the applicator member is secured to a reservoir of anhydrous composition to be applied to the hair to be treated, the reservoir preferably being made of the same material as the applicator member, more preferably with a lower density;
- the applicator member and the reservoir, if appropriate, are impregnated with a volume of anhydrous composition greater than 6 ml, preferably greater than 10 ml, and/or less than 14 ml, preferably less than 11 ml;
- the applicator member has an elongate shape, with a rectangular or cruciform cross section, with a flat, pointed or rounded end that is intended to be in contact with the hair to be treated, and/or with one or two bevels;
- the anhydrous composition comprises one or more natural or synthetic oils;
- the natural oils are chosen from mineral oils, preferably chosen from mixtures of hydrocarbon-based oils derived from petroleum, volatile or non-volatile liquid paraffin, liquid petroleum jelly, polyolefins
- the natural oils are chosen from plant oils, preferably chosen from triglyceride plant oils;
- the synthetic oils are chosen from silicone oils, preferably from linear, branched or cyclic, volatile or non-volatile, optionally organomodified silicone oils;
- the anhydrous composition comprises one or more silicone oils, preferably in a content of between 0.05 and 99.9% by weight, relative to the total weight of the composition;
- the anhydrous composition also comprises one or more ingredients chosen from natural or synthetic waxes, cationic, nonionic, anionic or amphoteric non-silicone polymers, UV-screening agents, colorants, surfactants, pigments, preservatives, and fragrances;
- the refill comprises a body delimiting a cavity that is formed at least partially by two opposite walls and a bottom and opens toward the outside by way of an opening, said applicator member being partially received in said cavity and extending partially out of the cavity, through the opening.

According to another aspect, the invention relates to a cosmetic product refill article, comprising a closed package and at least one cosmetic product refill as described above, in the package.

The invention also relates to a hair treatment device, comprising:
- at least one holder, preferably an arm comprising a housing,
- a cosmetic product refill as described above, which is disposed in a removable manner in the housing.

Preferably, the device comprises at least one heating element.

Preferably, the device is a hair straightener, notably a straightening iron.

Preferably, the hair treatment device comprises two arms that are able to move relative to one another between a moved-together configuration for treating the hair and a spaced-apart configuration for inserting hair to be treated between said arms.

According to one variant, at least one of the arms, preferably each arm, comprises a heating element for the hair to be treated.

According to yet another aspect, the invention relates to a ready-to-assemble hair treatment assembly, comprising:
- a hair treatment device, comprising at least one arm provided with a heating element for the hair, the hair treatment device comprising, on one of these arms, a receiving housing suitable for receiving a cosmetic product refill as described above, and
- a cosmetic product refill article as described above, in all the combinations thereof.

According to another aspect, the invention also relates to a method for treating the hair, comprising the step of applying a cosmetic product as described above to the hair with the aid of the device according to the invention.

Preferably, the treatment is carried out by moving the device along the lock of hair, the arms of said device being held in the moved-together configuration during said movement.

The invention may be understood better from reading the following detailed description of nonlimiting illustrative embodiments thereof and from studying the appended drawing, in which:
- Figure 1 shows a perspective view of an example of a hair treatment device,
- Figure 2 is a cross-sectional view on II-II of the hair treatment device in figure 1,
- Figure 3 illustrates, in cross section, examples of applicator members that can be employed in the hair treatment device in figure 1,
- Figure 4 illustrates tests of the quantity of cosmetic product delivered by different types of applicator member; and
- Figure 5 schematically illustrates a ready-to-assemble hair treatment assembly.

In the rest of the description, identical elements or elements having identical functions bear the same reference signs. In order to make the present description concise, they are not described for each of the figures, only the differences between the different examples being described.

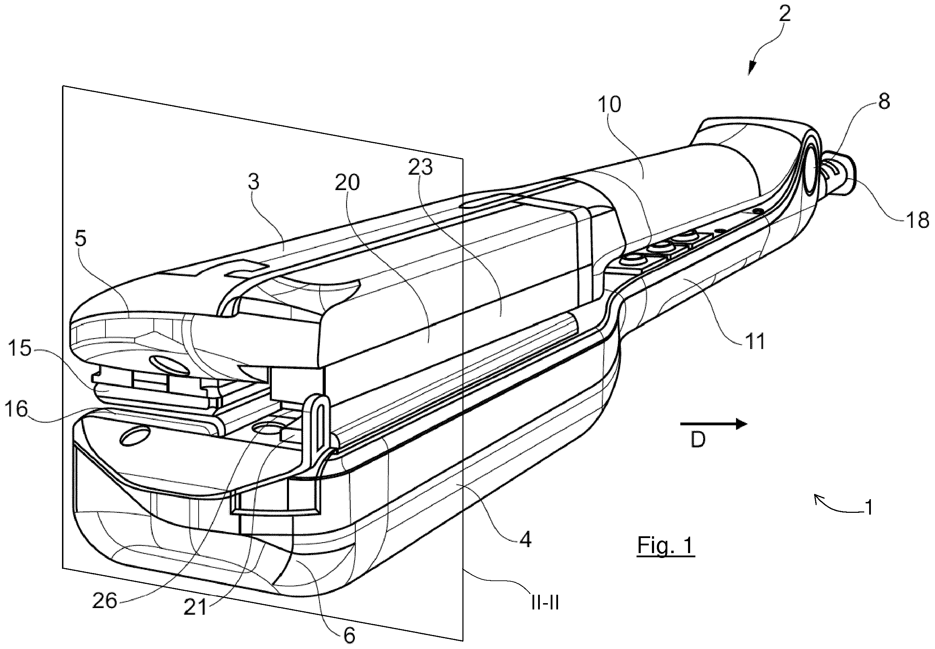

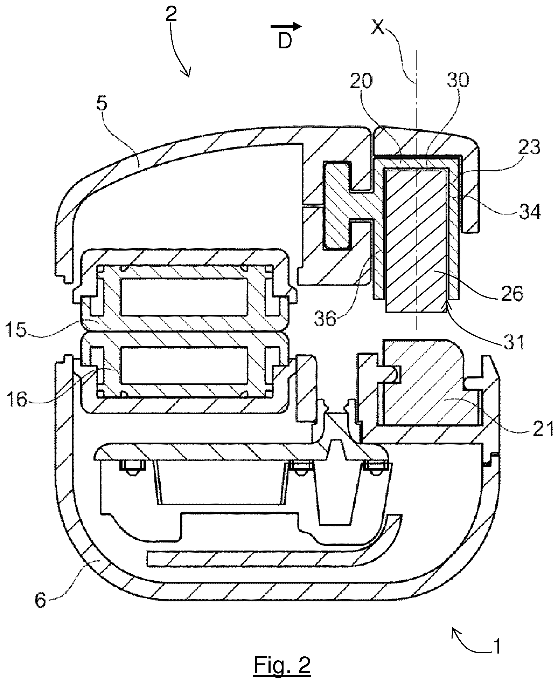

Figures 1 and 2 show the handpiece 2 of an example of a hair treatment device 1.

This handpiece 2 has two jaws 3 and 4 that are able to move with respect to one another between a spaced-apart configuration (not shown) for the introduction of a lock of hair between said jaws, and a moved-together configuration for treating the lock of hair.

The jaws 3 and 4 are carried by an upper arm 5 and a lower arm 6, respectively, which, in the example in question, are connected together at one end by an articulation 8, the handpiece 2 thus forming tongs.

The upper arm 5 and lower arm 6 each preferably have a total length of between 22 cm and 37 cm, preferably 31 cm, and define, between the articulation 8 and the jaws 3 and 4, respective half-handles 10 and 11 on which the user can press in order to move the jaws 3 and 4 together.

An elastic return member (not visible) is preferably provided to return the jaws 3 and 4 to a spaced-apart configuration, this elastic return member being for example a spring disposed around a pin of the articulation 8.

The invention is not limited to a particular manner of connecting the upper arm 5 and lower arm 6 together and the jaws 3 and 4 may be rendered able to move in some other way without departing from the scope of the present invention. However, the presence of an articulation is largely preferred for the ergonomics it provides.

The jaws 3 and 4 define between them a region for treating the hair, said region being intended to receive a lock of hair to be treated, the handpiece 2 being moved along said lock during the treatment, for example in the direction from the root to the end of the hair.

In the example in question, the handpiece 2 is configured to apply a cosmetic product according to the invention, to treat the hair by way of steam and then to carry out a heat treatment of the hair by contact with two hot surfaces of heating elements 15 and 16 that are carried by the upper arm 5 and the lower arm 6, respectively.

The direction D of movement of the handpiece 2 over the hair, illustrated in figure 1, is preferably substantially perpendicular to the upper arm 5 and lower arm 6.

The handpiece 2 is connected by a line, in the example in question, to a base station (not shown) that is fixed during the treatment and is connected to the mains.

This base station provides electric power to the handpiece 2 and also its supply of water for generating steam, and may also carry out additional functions of processing electrical signals received from the handpiece 2. The line 18 which connects the handpiece 2 to the base station may thus comprise various electrical conductors and a water supply pipe.

A user interface (not shown in the figures) may be present on the handpiece 2 so as to give the user the option for example of starting up certain components thereof.

The cosmetic product according to the invention is applied by a refill 20 carried by one of the two arms, 5 or 6, in this case the upper arm 5, which comes into contact with a pressing element 21. The latter may be removable.

The refill 20 comprises a body 23 and an applicator member 26 disposed so as to come into contact with the hair extending through the treatment region.

The refill 20 may be fastened to the arm 5 or 6 by any means, notably, as illustrated in figure 2, by sliding a rib of the body 23 into a groove with a complementary shape.

As illustrated in figure 2, the body 23 has a cavity 30 that opens toward the outside by way of an opening 31 into which the applicator member 26 is inserted, along an insertion axis X, while the refill is being manufactured. The body may comprise coupling reliefs that extend into the cavity 30.

The body 23 substantially has an elongate shape along a longitudinal axis. In cross section, the body 23 is U-shaped, as illustrated in figure 2.

As illustrated in figure 2, the applicator member 26 may be in the form of a rectangular parallelepiped. The applicator member 26 thus has a substantially flat face that is intended to be in contact with the hair to be treated and is parallel to the opposite surface of the applicator member 26. The dimensions of the applicator member 26 are thus, for example, 8 mm x 20 mm x 90 mm, the face intended to be in contact with the hair having dimensions of 8 mm x 90 mm.

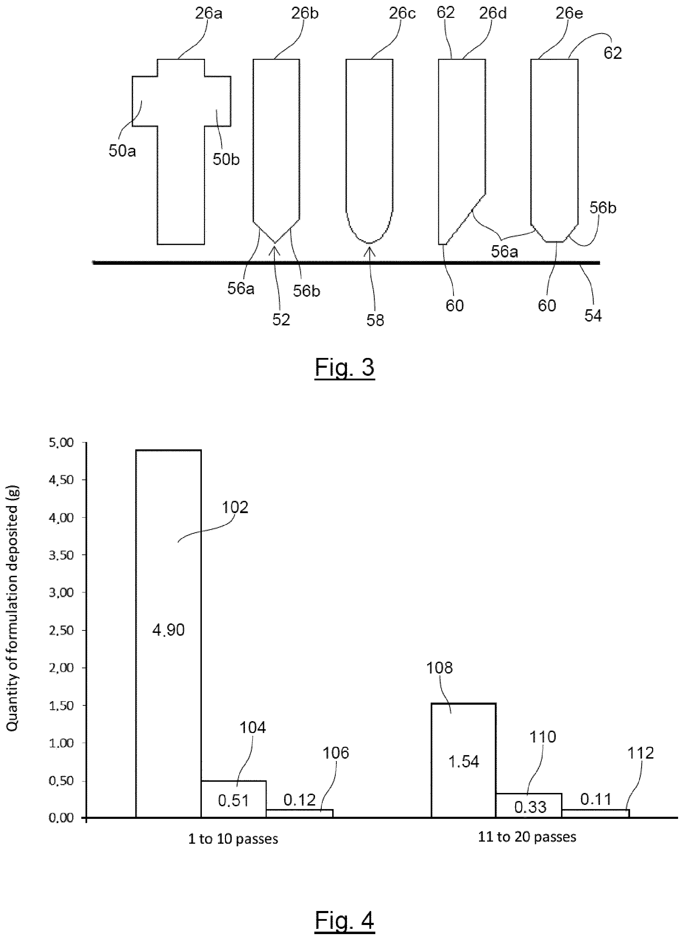

However, figure 3 illustrates possible variants of the applicator member 26. The applicator member 26a thus has a cruciform cross section, this applicator member 26a having, compared with the applicator member 26 in figure 2, two parallelepipedal longitudinal ribs 50a, 50b.

The applicator member 26b for its part has, compared with the applicator member 26 in figure 2, a point 52 intended to be in contact with the hair 54 to be treated. The point 52 is formed by two bevels 56a, 56b with opposite orientations.

The applicator member 26c has, in cross section, an ogival shape, the rounded end 58 of the ogive being intended to be oriented toward the hair 54.

The applicator member 26d has, compared with the applicator member 26b, a single bevel 56a. The applicator member 26d thus has a surface 60, intended to be in contact with the hair 54, that is parallel to the opposite surface 62 and has limited dimensions.

Finally, the applicator member 26e has two opposite bevels 56a, 56b that are connected by a central portion 60, intended to be in contact with the hair 54, which extends substantially parallel to the opposite surface 62 of the applicator member 26e.

Other applicator member shapes may also be envisioned, notably by combining the variants in figure 3.

The applicator member may be made for example of polyethylene, of polypropylene or of a mixture of polyethylene and polypropylene. The mixture may notably comprise 50% polyethylene and 50% polypropylene.

The applicator member may be made of felt.

A felt is, in particular, a nonwoven material obtained by agglutinating natural or synthetic fibers.

The fibers of the felt may be oriented parallel to the surface of the lock of hair 54 to be treated, the fibers preferably being oriented substantially in the direction D of passage over the hair. In one variant, the fibers of the felt of the applicator member are oriented perpendicularly to the surface of the lock of hair 54 to be treated, i.e. perpendicularly to the direction D and to the longitudinal direction of the applicator member 26.

The applicator member may comprise at least two parts having different fiber densities and/or fiber orientations. Notably, a part with a lower fiber density may act as a reservoir, while the other part, with a higher fiber density, allows more homogeneous application of cosmetic product to the lock of hair to be treated. In this way, the variation in quantity of product delivered onto one and the same lock is reduced, depending on the position on the lock.

The applicator member is for example impregnated with a volume of cosmetic product greater than 6 ml, preferably greater than 10 ml and/or less than 14 ml, preferably less than 11 ml. The applicator member has a density greater than 0.11 g/cm³ and less than 0.25 g/cm³.

In this case, the cosmetic product is an anhydrous composition. An "anhydrous composition" is understood here to be a composition containing less than 5% by weight of water, and more preferably less than 1% by weight of water, relative to the total weight of the composition. Preferably, the anhydrous composition does not contain any water added during the preparation of the composition, the only water present originating from the residual water provided by the mixed ingredients. Even more preferably, the anhydrous composition is free of water.

Preferably, the anhydrous composition comprises one or more oils.

The oils may be natural or synthetic oils.

The natural oils may be mineral oils or plant oils.

The mineral oils may be chosen from mixtures of hydrocarbon-based oils derived from petroleum, volatile or non-volatile liquid paraffin, liquid petroleum jelly, polyolefins.

The plant oils are preferably chosen from triglyceride plant oils.

The synthetic oils are preferably chosen from linear, branched or cyclic, volatile or non-volatile silicones. They may optionally be organomodified.

Silicone is understood to mean compounds comprising one or more atoms of silicon in their structure. The anhydrous composition may notably comprise a mixture of silicones.

When it contains any, the anhydrous composition comprises one or more oils in a content ranging from 1 to 100%, preferably from 5 to 99%, more preferably from 10 to 99%, better still from 25 to 99% and even better still from 50 to 99% by weight relative to the total weight of the composition.

The anhydrous composition according to the invention may also comprise one or more ingredients chosen from natural or synthetic waxes, cationic, nonionic, anionic or amphoteric non-silicone polymers, UV-screening agents, colorants, surfactants, pigments, preservatives, and fragrances.

The viscosity of the anhydrous composition is greater than 0.1 Pa.s (100 centipoise) and less than 0.6 Pa.s (600 centipoise), preferably less than 0.5 Pa.s (500 centipoise). The viscosity of the anhydrous composition is measured at a pressure of 1.01325 10⁵ Pa, at a temperature of 22°C and at a shear rate of 1 s⁻¹. This viscosity may be measured using a cone/plate viscometer, notably using a Haake R600 rheometer or a similar device.

After numerous tests, the inventors have found, surprisingly, that the combination of a density of the applicator member and a viscosity of the anhydrous composition as indicated above makes it possible to deliver a satisfactory quantity of anhydrous composition onto locks of hair to be treated, at least during the first passes of the applicator member over the locks of hair to be treated, in order to obtain a satisfactory cosmetic effect of the anhydrous composition on the hair. This is true notably for the first 70 passes, preferably for the first 75 passes, of the applicator member over locks of hair to be treated, these numbers corresponding to the treatment of an entire head. The passes of the applicator member are understood here as being passes at a substantially constant speed of 15 s over locks with a length of 27 cm and a width of 10.5 cm, with a force of 3 N being applied to the applicator member in the direction of the lock of hair to be treated.

The initial quantity of cosmetic product present in the applicator member preferably corresponds to an initial degree of filling of the applicator member less than or equal to 90%, preferably less than or equal to 80%, and more preferably less than or equal to 70%, and greater than or equal to 40%, preferably greater than or equal to 50%, and better still greater than or equal to 60%. The inventors have found that, when the applicator member is not completely saturated with cosmetic product but rather has a degree of filling as indicated above, the quantity of cosmetic product delivered on each pass over the hair to be treated exhibits less significant variations. On account of the use of refills according to the invention, it is possible to obtain a more regular, notably more homogeneous cosmetic effect on all of a user's hair. Of course, the consistency of the quantity of cosmetic product implies that the conditions of application of the cosmetic product, notably the speed of passage and the force of application, are kept substantially constant over the entire period of use of the hair treatment device.

The "initial quantity" is understood here to be the quantity of cosmetic product present in the applicator member before the first pass over the hair to be treated.

The "degree of filling of the applicator member" is understood here to be the ratio between the mass of cosmetic product effectively present in the applicator member and the maximum mass of cosmetic product with which the applicator member can be impregnated. This degree of filling is measured at atmospheric pressure of 1.01325 10⁵ Pa and a temperature of 22°C.

By way of example, figure 4 illustrates the application of particular anhydrous compositions with the aid of three different types of applicator member.

### Examples:

Composition A below was prepared from the ingredients indicated in the table below. The concentrations are expressed as weight percentages of active material in the composition.

| Ingredients | A |
|---|---|
| Cyclopentasiloxane | 94.6 |
| Dimethiconol | 5.4 |

This anhydrous composition has a viscosity of 0.113 Pa.s (113 centipoise) at 22°C.

The test to which the different applicator members are subjected consists in:
- carrying out ten passes of 15 seconds each of the applicator member over strips of paper of 75 g/m² IQ Appeal® from Mondigroup, each strip measuring 27 cm in length and 10.5 cm in width, a force of 3 N being applied to the applicator member, in the direction of the strip of paper, so as to deliver a first quantity of anhydrous composition onto the ten strips. This first quantity may be determined for example by weighing the applicator member before and after the ten passes, the total quantity of anhydrous composition deposited on the ten strips then corresponding to the difference between the masses measured;
- then, carrying out ten more passes of 15 seconds of the applicator member over new strips of paper of 75 g/m² IQ Appeal® from Mondigroup, each strip again measuring 27 cm in length and 10.5 cm in width, a force of 3 N being applied to the applicator member, in the direction of the strip of paper, so as to deliver a second quantity of anhydrous composition.

It should be noted here that it is important to saturate the strips along their entire length. In other words, the applicator member is moved over the strip at a speed of around 1.8 cm/s.

In all cases, the applicator members tested have a parallelepipedal shape with fibers oriented substantially in a longitudinal direction of the applicator member, parallel to the surface to which the anhydrous composition is applied. They are constituted of a felt comprising a mixture of 50% polyethylene and 50% polypropylene.

### 1^{st} sample tested:

The first sample tested comprises an applicator member with a density of 0.10 g/cm³ impregnated to 80% with the above anhydrous composition A.

### 2^{nd} sample tested:

The second sample differs from the first only by way of the density of the applicator member. It is equal to 0.18 g/cm³.

### 3^{rd} sample tested:

The third sample differs from the first and second only by way of the density of the applicator member, which is equal to 0.35 g/cm³.

Bars 102, 104 and 106 in figure 4 indicate the first quantity of anhydrous composition A deposited with the applicator member with a density of 0.1 g/cm³, 0.18 g/cm³ and 0.35 g/cm³, respectively. The corresponding values are 4.90 g, 0.51 g and 0.12 g.

It has been found that the best cosmetic results on hair, notably a better cosmetic feel and better homogeneity of deposition, were obtained when the two following criteria are met:
- a deposition of anhydrous composition per pass over strips of paper in the range from 0.2 g to 1.0 g
- the absolute value of the difference between the first quantity and the second quantity of anhydrous composition is less than or equal to 0.2 g.

Bars 108, 110 and 112 in figure 4 indicate the second quantity of anhydrous composition deposited with the applicator member with a density of 0.1 g/cm³, 0.18 g/cm³ and 0.35 g/cm³, respectively. The corresponding values are 1.54 g, 0.33 g and 0.11 g.

It has been found that the applicator member with a density of 0.35 g/cm³ meets the second criterion, but not the first. Therefore, it is not acceptable for the sought-after cosmetic result.

The applicator member with a density of 0.1 g/cm³ does not meet any of the abovementioned criteria. Therefore, it is not acceptable for the sought-after cosmetic result, either.

By contrast, the applicator member with a density of 0.18 g/cm³ meets both criteria. It proves to be effectively suitable for use that makes it possible to achieve a satisfactory cosmetic result on a user's hair.

The invention is not limited to the illustrative embodiments that have just been described, the features of which may be combined with one another within variants that are not illustrated.

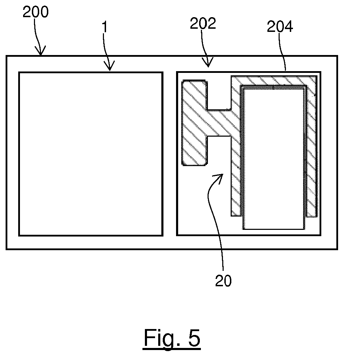

Thus, the invention relates, for example, to a ready-to-assemble assembly 200 as illustrated schematically in figure 5. This ready-to-assemble assembly 200 comprises a hair treatment device 1 and a cosmetic product refill article 202. This article comprises in this case a cosmetic product refill 20 in a package 204 which is closed so as to avoid, notably, any evaporation of the cosmetic product from the applicator member. In a variant, the package 204 may comprise a plurality of cosmetic product refills 20. In this case, it is preferred for the package to be able to be reclosed so that the other cosmetic product refills do not dry out when a refill is withdrawn from the package. In a variant, in this case, each individual refill is preserved in an individual package, inside a package common to the multiple refills.

Moreover, other examples of anhydrous compositions can be employed, as long as their density is between 0.1 and 0.6 Pa.s (100 and 600 centipoise).

## Claims

1. A cosmetic product refill (20) for a hair treatment device (1), the cosmetic product being an anhydrous composition, the cosmetic product comprising an applicator member (26) impregnated with the anhydrous composition, wherein the applicator member has a density of between 0.11 and 0.25 g/cm³ and the anhydrous composition has a viscosity of between 0.1 and 0.6 Pa.s (100 and 600 centipoise), preferably between 0.1 and 0.5 Pa.s (100 and 500 centipoise), measured at a pressure of 1.01325 10⁵ Pa, at a temperature of 22°C and at a shear rate of 1 s⁻¹, the applicator member (26) being preferably porous.

2. The cosmetic product refill as claimed in claim 1, wherein the applicator member (26) has a density of between 0.15 and 0.2 g/cm³ and the anhydrous composition has a viscosity greater than 0.1 Pa.s (100 centipoise), preferably greater than 0.11 Pa.s (110 centipoise) and/or less than 0.12 Pa.s (120 centipoise), preferably less than 0.115 Pa.s (115 centipoise), at 22°C.

3. The cosmetic product refill as claimed in claim 1 or 2, wherein the applicator member (26) is a felt, the fibers of the applicator member preferably being oriented parallel or perpendicularly to the surface intended to be in contact with the hair to be treated.

4. The cosmetic product refill as claimed in any one of the preceding claims, wherein the applicator member (26), notably the felt, if appropriate, is made of polyethylene, of polypropylene, or of a mixture of polyethylene and polypropylene, the mixture preferably comprising 50% polyethylene and 50% polypropylene.

5. The cosmetic product refill as claimed in any one of the preceding claims, wherein the applicator member (26) is impregnated with anhydrous composition with an initial degree of filling less than or equal to 90%, preferably less than or equal to 80%, more preferably less than or equal to 70%, and/or greater than or equal to 40%, preferably greater than or equal to 50%, more preferably greater than or equal to 60%.

6. The cosmetic product refill as claimed in one of the preceding claims, wherein the applicator member (26) is secured to a reservoir of anhydrous composition to be applied to the hair to be treated, the reservoir preferably being made of the same material as the applicator member, more preferably with a lower density, preferably the applicator member (26) and the reservoir, if appropriate, being impregnated with a volume of anhydrous composition greater than 6 ml, preferably greater than 10 ml, and/or less than 14 ml, preferably less than 11 ml.

7. The cosmetic product refill as claimed in any one of the preceding claims, wherein the applicator member (26) has an elongate shape, with a rectangular or cruciform cross section, with a flat, pointed or rounded end that is intended to be in contact with the hair to be treated, and/or with one or two bevels.

8. The cosmetic product refill as claimed in any one of the preceding claims, wherein the anhydrous composition comprises one or more natural or synthetic oils, preferably the anhydrous composition comprising one or more silicone oils, preferably in a content of between 0.05 and 99.9% by weight, relative to the total weight of the composition.

9. The cosmetic product refill as claimed in the preceding claim, wherein the natural oils are chosen from mineral oils, preferably chosen from mixtures of hydrocarbon-based oils derived from petroleum, volatile or non-volatile liquid paraffin, liquid petroleum jelly, polyolefins, or from plant oils, preferably chosen from triglyceride plant oils.

10. The cosmetic product refill as claimed in claim 8, wherein the synthetic oils are chosen from silicone oils, preferably from linear, branched or cyclic, volatile or non-volatile, optionally organomodified silicone oils.

11. The cosmetic product refill as claimed in any one of the preceding claims, wherein the anhydrous composition also comprises one or more ingredients chosen from natural or synthetic waxes, cationic, nonionic, anionic or amphoteric non-silicone polymers, UV-screening agents, colorants, surfactants, pigments, preservatives, and fragrances.

12. The cosmetic product refill as claimed in any one of the preceding claims, comprising a body delimiting a cavity that is formed at least partially by two opposite walls and a bottom and opens toward the outside by way of an opening, said applicator member (26) being partially received in said cavity and extending partially out of the cavity, through the opening.

13. A cosmetic product refill article comprising a closed package and at least one cosmetic product refill as claimed in any one of the preceding claims, in the package.

14. A hair treatment device (1), being preferably a hair straightener, notably a straightening iron, comprising:
- at least one holder, preferably an arm (5, 6) comprising a housing,
- a cosmetic product refill (20) as claimed in any one of claims 1 to 10, which is disposed in a removable manner in the housing,
the device preferably comprising one heating element.

15. A method for treating the hair, comprising the step of applying a cosmetic product as defined in claims 1-11 to the hair using a device as claimed in claim 14.

## Patentansprüche

1. Nachfüllung (20) eines kosmetischen Produkts für eine Haarbehandlungsvorrichtung (1), wobei das kosmetische Produkt eine wasserfreie Zusammensetzung ist, wobei das kosmetische Produkt ein Applikatorelement (26) umfasst, welches mit der wasserfreien Zusammensetzung imprägniert ist, wobei das Applikatorelement eine Dichte zwischen 0,11 und 0,25 g/cm³ aufweist und die wasserfreie Zusammensetzung eine Viskosität zwischen 0,1 und 0,6 Pa.s (100 und 600 Centipoise) aufweist, bevorzugt zwischen 0,1 und 0,5 Pa.s (100 und 500 Centipoise), gemessen bei einem Druck von 1,01325 10⁵ Pa, bei einer Temperatur von 22°C und einer Schergeschwindigkeit von 1 s⁻¹, wobei das Applikatorelement (26) bevorzugt porös ist.

2. Nachfüllung des kosmetischen Produkts nach Anspruch 1, wobei das Applikatorelement (26) eine Dichte von 0,15 und 0,2 g/cm³ aufweist und die wasserfreie Zusammensetzung eine Viskosität größer als 0,1 Pa.s (100 Centipoise), bevorzugt größer als 0,11 Pa.s (110 Centipoise) und/oder weniger als 0,12 Pa.s (120 Centipoise), bevorzugt weniger als 0,115 Pa.s (115 Centipoise), bei 22°C aufweist.

3. Nachfüllung des kosmetischen Produkts nach Anspruch 1 oder 2, wobei das Applikatorelement (26) ein Filz ist, wobei die Fasern des Applikatorelements bevorzugt parallel oder senkrecht zu der Oberfläche ausgerichtet sind, welche mit den zu behandelnden Haaren in Kontakt sein soll.

4. Nachfüllung des kosmetischen Produkts nach einem der vorhergehenden Ansprüche, wobei das Applikatorelement (26), insbesondere der Filz, falls zutreffend, aus Polyethylen, aus Polypropylen oder einer Mischung aus Polyethylen und Polypropylen gemacht ist, wobei die Mischung bevorzugt 50% Polyethylen und 50% Polypropylen umfasst.

5. Nachfüllung des kosmetischen Produkts nach einem der vorhergehenden Ansprüche, wobei das Applikatorelement (26) mit der wasserfreien Zusammensetzung mit einem anfänglichen Füllgrad von weniger als oder gleich 90%, bevorzugt weniger als oder gleich 80%, bevorzugter weniger als oder gleich 70% und/oder größer als oder gleich 40%, bevorzugt größer als oder gleich 50%, bevorzugter größer als oder gleich 60%, imprägniert ist.

6. Nachfüllung des kosmetischen Produkts nach einem der vorhergehenden Ansprüche, wobei das Applikatorelement (26) an einem Reservoir an wasserfreier Zusammensetzung befestigt ist, welche auf die zu behandelnden Haare aufgebracht werden soll, wobei das Reservoir bevorzugt aus demselben Material wie das Applikatorelement gemacht ist, bevorzugter mit einer geringeren Dichte, wobei das Applikatorelement (26) und das Reservoir bevorzugt, falls zutreffend, mit einem Volumen der wasserfreien Zusammensetzung größer als 6 ml, bevorzugt größer als 10 ml, und/oder weniger als 14 ml, bevorzugt weniger als 11 ml, imprägniert sind.

7. Nachfüllung des kosmetischen Produkts nach einem der vorhergehenden Ansprüche, wobei das Applikatorelement (26) eine längliche Form mit einem rechteckigen oder kreuzförmigen Querschnitt, mit einem flachen, spitzen oder abgerundeten Ende aufweist, welches in Kontakt mit dem zu behandelnden Haaren sein soll, und/oder mit einer oder zwei Fasen.

8. Nachfüllung des kosmetischen Produkts nach einem der vorhergehenden Ansprüche, wobei die wasserfreie Zusammensetzung ein oder mehrere natürliche oder synthetische Öle umfasst, wobei die wasserfreie Zusammensetzung bevorzugt ein oder mehrere Silikonöle umfasst, bevorzugt in einem Gehalt zwischen 0,05 und 99,9 Gewichtsprozent bezogen auf das Gesamtgewicht der Zusammensetzung.

9. Nachfüllung des kosmetischen Produkts nach einem der vorhergehenden Ansprüche, wobei die natürlichen Öle ausgewählt sind aus Mineralölen, bevorzugt ausgewählt aus Mischungen von Ölen auf Kohlenwasserstoffbasis abgeleitet von Petroleum, flüchtigen oder nichtflüchtigen flüssigen Paraffin, flüssiger Vaseline, Polyolefinen oder aus Pflanzenölen, bevorzugt ausgewählt aus Triglycerid-Pflanzenölen.

10. Nachfüllung des kosmetischen Produkts nach Anspruch 8, wobei die synthetischen Öle ausgewählt sind aus Silikonölen, bevorzugt aus linearen, verzweigten oder zyklischen, flüchtigen oder nichtflüchtigen, optional organomodifizierten Silikonölen.

11. Nachfüllung des kosmetischen Produkts nach einem der vorhergehenden Ansprüche, wobei die wasserfreie Zusammensetzung zudem einen oder mehrere Bestandteile ausgewählt aus natürlichen oder synthetischen Wachsen, kationischen, nichtionischen, anionischen oder amphoteren Nicht-Silikonpolymeren, UV-Filtermitteln, Farbstoffen, Tensiden, Pigmenten, Konservierungsmitteln und Duftstoffen umfasst.

12. Nachfüllung des kosmetischen Produkts nach einem der vorhergehenden Ansprüche, umfassend einen Körper, der einen Hohlraum begrenzt, der zumindest teilweise aus zwei gegenüberliegenden Wänden und einen Boden gebildet ist und sich über eine Öffnung nach außen öffnet, wobei das Applikatorelement (26) teilweise in dem Hohlraum aufgenommen ist und sich teilweise aus dem Hohlraum heraus durch die Öffnung erstreckt.

13. Nachfüllartikel eines kosmetischen Produkts, umfassend eine geschlossene Verpackung und zumindest eine Nachfüllung des kosmetischen Produkt nach einem der vorhergehenden Ansprüche in der Verpackung.

14. Haarbehandlungsvorrichtung (1), welche bevorzugt ein Haarglätter, insbesondere ein Glätteisen ist, umfassend:
- zumindest einen Halter, bevorzugt einen Arm (5, 6), welcher ein Gehäuse umfasst,
- eine Nachfüllung (20) eines kosmetischen Produkts nach einem der Ansprüche 1 bis 10, wobei diese entfernbar in dem Gehäuse angeordnet ist,
wobei die Vorrichtung bevorzugt zumindest ein Heizelement umfasst.

15. Verfahren zum Behandeln von Haaren, umfassend den Schritt des Aufbringens eines kosmetischen Produkts, wie in den Ansprüchen 1 bis 11 definiert, auf die Haare unter Verwendung einer Vorrichtung gemäß Anspruch 14.

## Revendications

1. Recharge de produit cosmétique (20) pour dispositif de traitement de la chevelure (1), le produit cosmétique étant une composition anhydre, le produit cosmétique comprenant un organe d'application (26) imbibé de la composition anhydre, dans laquelle l'organe d'application a une masse volumique comprise entre 0,11 et 0,25 g/cm³ et la composition anhydre a une viscosité comprise entre 0,1 et 0,6 Pa.s (entre 100 et 600 centipoises), de préférence entre 0,1 et 0,5 Pa.s (entre 100 et 500 centipoises), mesurée sous une pression de 1,01325 10⁵ Pa, à une température de 22°C et à une vitesse de cisaillement de 1 s⁻¹, l'organe d'application (26) étant de préférence poreux.

2. Recharge de produit cosmétique selon la revendication 1, dans laquelle l'organe d'application (26) a une masse volumique comprise entre 0,15 et 0,2 g/cm³ et la composition anhydre a une viscosité supérieure à 0,1 Pa.s (100 centipoises), de préférence supérieure à 0,11 Pa.s (110 centipoises), et/ou inférieure à 0,12 Pa.s (120 centipoises), de préférence inférieure à 0,115 Pa.s (115 centipoises), à 22 °C.

3. Recharge de produit cosmétique selon la revendication 1 ou 2, dans laquelle l'organe d'application (26) est un feutre, les fibres de l'organe d'application étant de préférence orientées parallèlement ou perpendiculairement à la surface destinée à être en contact avec les cheveux à traiter.

4. Recharge de produit cosmétique selon l'une quelconque des revendications précédentes, dans laquelle l'organe d'application (26), notamment le feutre le cas échéant, est en polyéthylène, en polypropylène, ou en un mélange de polyéthylène et de polypropylène, le mélange comportant de préférence 50 % de polyéthylène et 50 % de polypropylène.

5. Recharge de produit cosmétique selon l'une quelconque des revendications précédentes, dans laquelle l'organe d'application (26) est imbibé de composition anhydre avec un taux initial de remplissage inférieur ou égal à 90 %, de préférence inférieur ou égal à 80 %, de préférence encore inférieur ou égal à 70 %, et/ou supérieur ou égal à 40 %, de préférence supérieur ou égal à 50 %, de préférence encore supérieur ou égal à 60 %.

6. Recharge de produit cosmétique selon l'une quelconque des revendications précédentes, dans laquelle l'organe d'application (26) est solidaire d'un réservoir de composition anhydre à appliquer sur les cheveux à traiter, le réservoir étant de préférence réalisé dans le même matériau que l'organe d'application, de préférence encore avec une densité inférieure, de préférence l'organe d'application (26) et le réservoir, le cas échéant, étant imbibés d'un volume de composition anhydre supérieur à 6 ml, de préférence supérieur à 10 ml, et/ou inférieur à 14 ml, de préférence inférieur à 11 ml.

7. Recharge de produit cosmétique selon l'une quelconque des revendications précédentes, dans laquelle l'organe d'application (26) a une forme allongée, de section transversale rectangulaire ou en croix, avec une extrémité destinée à être en contact avec les cheveux à traiter, plane, pointue ou arrondie, et/ou avec un ou deux biseaux.

8. Recharge de produit cosmétique selon l'une quelconque des revendications précédentes, dans laquelle la composition anhydre comprend une ou plusieurs huiles naturelles ou synthétiques, de préférence la composition anhydre comprenant une ou plusieurs huiles de silicone, de préférence dans une teneur comprise entre 0,05 et 99,9 % en poids par rapport au poids total de la composition.

9. Recharge de produit cosmétique selon la revendication précédente dans laquelle les huiles naturelles sont choisies parmi les huiles minérales, de préférence choisies parmi les mélanges d'huiles hydrocarbonées dérivées du pétrole, la paraffine liquide volatile ou non volatile, l'huile de vaseline, les polyoléfines, ou parmi les huiles végétales, de préférence choisies parmi les huiles triglycéridiques végétales.

10. Recharge de produit cosmétique selon la revendication 8, dans laquelle les huiles synthétiques sont choisies parmi les huiles de silicone, de préférence parmi les huiles de silicone linéaires, ramifiées ou cycliques, volatiles ou non, éventuellement organomodifiées.

11. Recharge de produit cosmétique selon l'une quelconque des revendications précédentes, dans laquelle la composition anhydre comprend en outre un ou plusieurs ingrédients choisis parmi les cires naturelles ou synthétiques, les polymères non siliconés cationiques, non ioniques, anioniques ou amphotères, les filtres anti UV, les colorants, les tensioactifs, les pigments, les conservateurs et les parfums.

12. Recharge de produit cosmétique selon l'une quelconque des revendications précédentes, comprenant un corps délimitant une cavité formée au moins partiellement par deux parois opposées et un fond, et s'ouvrant vers l'extérieur par une ouverture, ledit organe d'application (26) étant partiellement reçu dans ladite cavité et s'étendant en partie hors de la cavité, à travers l'ouverture.

13. Article de recharge(s) de produit cosmétique, comprenant un emballage fermé et au moins une recharge de produit cosmétique selon l'une quelconque des revendications précédentes, dans l'emballage.

14. Dispositif de traitement de la chevelure (1), qui est de préférence un lisseur à cheveux, notamment un fer à lisser, comportant :
- au moins un support de préférence un bras (5, 6) comportant un logement,
- une recharge de produit cosmétique (20) selon l'une quelconque des revendications 1 à 10 disposée de façon amovible dans le logement,
le dispositif comportant au moins un élément chauffant.

15. Procédé de traitement de la chevelure comportant l'étape consistant à appliquer un produit cosmétique tel que défini dans les revendications 1 à 11 sur les cheveux à l'aide d'un dispositif selon la revendication 14.
